# EUROPEAN PATENT APPLICATION

(11) **EP 2 267 141 A1**
(43) Date of publication of application: **29.12.2010**
(21) Application number: 09163891.6
(22) Date of filing: 26.06.2009
(51) Int. Cl.: C12P 7/16, C12N 15/09, C12N 1/15

(54) **Process for the biological production of n-Butanol with high yield**

(71) Applicant: Metabolic Explorer, 63360 Saint Beauzire (FR)
(72) Inventor: Soucaille, Philippe, 31450, DEYME (FR)
(74) Representative: Tetaz, Franck Claude Edouard

(57) **Abstract**

The present invention provides a method for the biological production of n-butanol at high yield from a fermentable carbon source. In one aspect of the present invention, a process for the conversion of glucose to n-butanol is achieved by the use of a recombinant organism comprising a host *C*. *acetobutlicum* transformed i) to eliminate the acetate pathway ii) to eliminate the butyrate pathway iii) to eliminate the lactate pathway and iv) to eliminate the acetone pathway. In another aspect of the present invention, the hydrogen flux is decreased and the reducing power redirected to n-butanol production by interrupting the expression of the hydrogenase gene. Optionally the n-butanol produced can be eliminated during the fermentation by gas striping and further purified by distillation.

SEQUENCE LISTING

<110>  METABOLIC EXPLORER
       SOUCAILLE, PHILIPPE

<120>  PROCESS FOR THE BIOLOGICAL PRODUCTION OF N-BUTANOL WITH HIGH
       YIELD

<130>  D26937

<160>  49

<170>  PatentIn version 3.5

<210>  1
<211>  53
<212>  DNA
<213>  Artificial Sequence

<220>
<223>  Oligonucleotide

<400>  1
aaaaaagctt ataattatcc ttagtactcg ctaaagtgcg cccagatagg gtg          53

<210>  2
<211>  60
<212>  DNA
<213>  Artificial Sequence

<220>
<223>  Oligonucleotide

<400>  2
cagattgtac aaatgtggtg ataacagata agtcgctaaa ggtaacttac ctttctttgt    60

<210>  3
<211>  49
<212>  DNA
<213>  Artificial Sequence

<220>
<223>  Oligonucleotide

<400>  3
tgaacgcaag tttctaattt cgattagtac tcgatagagg aaagtgtct               49

<210>  4
<211>  53
<212>  DNA
<213>  Artificial Sequence

<220>
<223>  Oligonucleotide

<400>  4
aaaaaagctt ataattatcc ttatctaaca caagcgtgcg cccagatagg gtg          53

<210>  5
<211>  60
<212>  DNA
<213>  Artificial Sequence

<220>
<223>  Oligonucleotide

<400>  5
cagattgtac aaatgtggtg ataacagata agtcacaagc tttaacttac ctttctttgt      60


<210>  6
<211>  49
<212>  DNA
<213>  Artificial Sequence

<220>
<223>  Oligonucleotide

<400>  6
tgaacgcaag tttctaattt cggttttaga tcgatagagg aaagtgtct                  49


<210>  7
<211>  53
<212>  DNA
<213>  Artificial Sequence

<220>
<223>  Oligonucleotide

<400>  7
aaaaaagctt ataattatcc ttagaagaca aaagagtgcg cccagatagg gtg             53


<210>  8
<211>  60
<212>  DNA
<213>  Artificial Sequence

<220>
<223>  Oligonucleotide

<400>  8
cagattgtac aaatgtggtg ataacagata agtcaaaaga aataacttac ctttctttgt      60


<210>  9
<211>  49
<212>  DNA
<213>  Artificial Sequence

<220>
<223>  Oligonucleotide

<400>  9
tgaacgcaag tttctaattt cgatttcttc tcgatagagg aaagtgtct                  49


<210>  10
<211>  53
<212>  DNA
<213>  Artificial Sequence

<220>
<223>  Oligonucleotide

<400>  10
aaaaaagctt ataattatcc ttatgtaccc aatgtgtgcg cccagatagg gtg             53


<210>  11
<211>  60
<212>  DNA
<213>  Artificial Sequence

<220>

<223>  Oligonucleotide

<400>  11
cagattgtac aaatgtggtg ataacagata agtccaatgt tttaacttac ctttctttgt      60


<210>  12
<211>  49
<212>  DNA
<213>  Artificial Sequence

<220>
<223>  Oligonucleotide

<400>  12
tgaacgcaag tttctaattt cggttataca tcgatagagg aaagtgtct                  49


<210>  13
<211>  53
<212>  DNA
<213>  Artificial Sequence

<220>
<223>  Oligonucleotide

<400>  13
aaaaaagctt ataattatcc ttacaactca aattggtgcg cccagatagg gtg             53


<210>  14
<211>  60
<212>  DNA
<213>  Artificial Sequence

<220>
<223>  Oligonucleotide

<400>  14
cagattgtac aaatgtggtg ataacagata agtcaaattg gttaacttac ctttctttgt      60


<210>  15
<211>  49
<212>  DNA
<213>  Artificial Sequence

<220>
<223>  Oligonucleotide

<400>  15
tgaacgcaag tttctaattt cggttagttg tcgatagagg aaagtgtct                  49


<210>  16
<211>  53
<212>  DNA
<213>  Artificial Sequence

<220>
<223>  Oligonucleotide

<400>  16
aaaaaagctt ataattatcc ttatgtatcc tggaagtgcg cccagatagg gtg             53


<210>  17
<211>  60
<212>  DNA
<213>  Artificial Sequence

<220>
<223>  Oligonucleotide

<400>  17
cagattgtac aaatgtggtg ataacagata agtcctggaa cataacttac ctttctttgt      60


<210>  18
<211>  49
<212>  DNA
<213>  Artificial Sequence

<220>
<223>  Oligonucleotide

<400>  18
tgaacgcaag tttctaattt cggttataca tcgatagagg aaagtgtct                  49


<210>  19
<211>  53
<212>  DNA
<213>  Artificial Sequence

<220>
<223>  Oligonucleotide

<400>  19
aaaaaagctt ataattatcc ttacaagacg agccagtgcg cccagatagg gtg             53


<210>  20
<211>  60
<212>  DNA
<213>  Artificial Sequence

<220>
<223>  Oligonucleotide

<400>  20
cagattgtac aaatgtggtg ataacagata agtcgagcca gttaacttac ctttctttgt      60


<210>  21
<211>  49
<212>  DNA
<213>  Artificial Sequence

<220>
<223>  Oligonucleotide

<400>  21
tgaacgcaag tttctaattt cggtttcttg tcgatagagg aaagtgtct                  49


<210>  22
<211>  53
<212>  DNA
<213>  Artificial Sequence

<220>
<223>  Oligonucleotide

<400>  22
aaaaaagctt ataattatcc ttataggtcg tgtacgtgcg cccagatagg gtg             53


<210>  23
<211>  60

<212>  DNA
<213>  Artificial Sequence

<220>
<223>  Oligonucleotide

<400>  23
cagattgtac aaatgtggtg ataacagata agtcgtgtac tataacttac ctttctttgt      60


<210>  24
<211>  49
<212>  DNA
<213>  Artificial Sequence

<220>
<223>  Oligonucleotide

<400>  24
tgaacgcaag tttctaattt cggttaccta tcgatagagg aaagtgtct      49


<210>  25
<211>  53
<212>  DNA
<213>  Artificial Sequence

<220>
<223>  Oligonucleotide

<400>  25
aaaaaagctt ataattatcc ttaatatacg gattagtgcg cccagatagg gtg      53


<210>  26
<211>  60
<212>  DNA
<213>  Artificial Sequence

<220>
<223>  Oligonucleotide

<400>  26
cagattgtac aaatgtggtg ataacagata agtcggatta aataacttac ctttctttgt      60


<210>  27
<211>  49
<212>  DNA
<213>  Artificial Sequence

<220>
<223>  Oligonucleotide

<400>  27
tgaacgcaag tttctaattt cggtttatat ccgatagagg aaagtgtct      49


<210>  28
<211>  53
<212>  DNA
<213>  Artificial Sequence

<220>
<223>  Oligonucleotide

<400>  28
aaaaaagctt ataattatcc ttacttgcct ctgaggtacg cccagatagg gtg      53

<210> 29
<211> 60
<212> DNA
<213> Artificial Sequence

<220>
<223> Oligonucleotide

<400> 29
cagattgtac aaatgtggtg ataacagata agtctctgag attaacttac ctttctttgt 60


<210> 30
<211> 49
<212> DNA
<213> Artificial Sequence

<220>
<223> Oligonucleotide

<400> 30
tgaacgcaag tttctaattt cggttgcaag tcgatagagg aaagtgtct 49


<210> 31
<211> 53
<212> DNA
<213> Artificial Sequence

<220>
<223> Oligonucleotide

<400> 31
aaaaaagctt ataattatcc ttacccatct acacggtgcg cccagatagg gtg 53


<210> 32
<211> 60
<212> DNA
<213> Artificial Sequence

<220>
<223> Oligonucleotide

<400> 32
cagattgtac aaatgtggtg ataacagata agtctacacg tctaacttac ctttctttgt 60


<210> 33
<211> 49
<212> DNA
<213> Artificial Sequence

<220>
<223> Oligonucleotide

<400> 33
tgaacgcaag tttctaattt cggttatggg tcgatagagg aaagtgtct 49


<210> 34
<211> 53
<212> DNA
<213> Artificial Sequence

<220>
<223> Oligonucleotide

<400> 34
aaaaaagctt ataattatcc ttaattacca tccttgtgcg cccagatcgg gtg 53

<210>  35
<211>  60
<212>  DNA
<213>  Artificial Sequence

<220>
<223>  Oligonucleotide

<400>  35
cagattgtac aaatgtggtg ataacagata agtcatcctt gataacttac ctttctttgt      60


<210>  36
<211>  49
<212>  DNA
<213>  Artificial Sequence

<220>
<223>  Oligonucleotide

<400>  36
tgaacgcaag tttctaattt cggttgtaat ccgatagagg aaagtgtct                 49


<210>  37
<211>  53
<212>  DNA
<213>  Artificial Sequence

<220>
<223>  Oligonucleotide

<400>  37
aaaaaagctt ataattatcc ttatatctcc tggtagtgcg cccagatagg gtg            53


<210>  38
<211>  60
<212>  DNA
<213>  Artificial Sequence

<220>
<223>  Oligonucleotide

<400>  38
cagattgtac aaatgtggtg ataacagata agtcctggta aataacttac ctttctttgt      60


<210>  39
<211>  49
<212>  DNA
<213>  Artificial Sequence

<220>
<223>  Oligonucleotide

<400>  39
tgaacgcaag tttctaattt cggttagata tcgatagagg aaagtgtct                 49


<210>  40
<211>  46
<212>  DNA
<213>  Artificial Sequence

<220>
<223>  Oligonucleotide

<400>  40
aaataatgga tccagagaca tcactaaaaa cagaaacata tgctag                    46


<210>  41
<211>  33
<212>  DNA
<213>  Artificial Sequence

<220>
<223>  Oligonucleotide

<400>  41
gaaccttctg cacttatatc cattggtatt ccc                                  33


<210>  42
<211>  26
<212>  DNA
<213>  Artificial Sequence

<220>
<223>  Oligonucleotide

<400>  42
tagtaaaagg gagtgtacga ccagtg                                          26


<210>  43
<211>  25
<212>  DNA
<213>  Artificial Sequence

<220>
<223>  Oligonucleotide

<400>  43
cgatttcgtc atggtctcca acaag                                           25


<210>  44
<211>  33
<212>  DNA
<213>  Artificial Sequence

<220>
<223>  Oligonucleotide

<400>  44
gcttcatata taggcagcaa cccagatact ggc                                  33


<210>  45
<211>  29
<212>  DNA
<213>  Artificial Sequence

<220>
<223>  Oligonucleotide

<400>  45
   ccacccatac cagagagcat ttttccagg                                        29


<210>  46
<211>  35
<212>  DNA
<213>  Artificial Sequence

<220>

<223>  Oligonucleotide

<400>  46
ggaacctcta taatatcctt cactccgtta attcc                                35


<210>  47
<211>  31
<212>  DNA
<213>  Artificial Sequence

<220>
<223>  Oligonucleotide

<400>  47
ggatttgttg gttcttctac agtatttgcg c                                    31


<210>  48
<211>  24
<212>  DNA
<213>  Artificial Sequence

<220>
<223>  Oligonucleotide

<400>  48
ggtattgaat attcgaaatc aacc                                            24


<210>  49
<211>  26
<212>  DNA
<213>  Artificial Sequence

<220>
<223>  Oligonucleotide

<400>  49
gctgataaat atgtagctga acctgg                                          26

## Description

### FIELD OF INVENTION

The invention comprises a process for the bioconversion of a fermentable carbon source to n-butanol at high yield by a metabolically engineered microorganism.

### BACKGROUND OF THE INVENTION

n-Butanol is a colorless, neutral liquid of medium volatility with restricted miscibility (about 7-8%) in water, but freely miscible with all common solvents such as glycols, ketones, alcohol, aldehydes, ethers, and aromatic and aliphatic hydrocarbons. n-Butanol is used i) to make other chemicals, ii) as a solvent and iii) as an ingredient in formulated products such as cosmetics. The major uses of n-butanol as a feed-stock are in the synthesis of acrylate/methacrylate esters, glycol ethers, n-Butyl acetate, amino resins and n-Butylamines. Currently, more than 9 millions tons of n-Butanol are consumed annually in the world.

More recently it has been shown that n-butanol is a better biofuel than ethanol due to lower vapour pressure, higher energy content (closer to that of gasoline) and lesser susceptibility to separation in the presence of water. Furthermore, n-butanol can be blended at higher concentrations than ethanol for use in standard vehicle engines and it does not require automakers to compromise on performance to meet environmental regulations. It is also suitable for transport in pipelines and as a result it has the potential to be introduced into gasoline quickly and avoid the need for additional large-scale supply infrastructures.

n-butanol can be produced as an acetone/n-butanol/ethanol (ABE) mixture by the fermentation of carbohydrate by solventogenic *Clostridia.* The ABE fermentations are biphasic. During the first acidogenic phase, high growth rate is accompanied by acetic and butyric acids production. In the second solventogenic phase growth rate decrease and the solvents (ABE) are produced with the concomitant consumption of the organic acids produced in the first phase. Carbon dioxide and hydrogen are produced throughout the fermentation.

The biological production of n-butanol requires the formation of butyryl-CoA as an intermediate which can be reduced, depending on the physiological conditions, by two different bi-functional aldehyde-alcohol dehydrogenases encoded by *adhE1* and *adhE2.* Butyryl-CoA can also be converted to butyric acid by a phospho-transbutyrylase and a butyrate kinase encoded respectively by the *ptb* and *buk* genes. Acetate is produced from acetyl-CoA by a phosphotransacetylase encoded by the gene *pta* and by an acetate kinase encoded by the gene *ack.* Acetone is produced from aceto-acetyl-CoA (an intermediate in the production of butyryl-CoA) by a CoA-transferase and an acetoacetate decarboxylase encoded respectively by the *ctfAB* and *adc* genes. Hydrogen is produced by an iron only hydrogenase encoded by the *hydA* gene. When cultures are performed in the presence of carbon monoxide, a hydrogenase inhibitor, n-butanol, ethanol and lactate are the main fermentation products. Lactate is produced from pyruvate by a lactate dehydrogenase encoded by the *ldh* gene.

*Clostridium acetobutylicum* strains with an inactivated *buk* gene (obtained by single crossing over with a non-replicable plasmid) have already been described in the article (Green et al., 1996). The non-replicable vector pJC4BK, with a 0.8 kb internal *buk* fragment was integrated into the chromosomal *buk gene* which leaded to an inactivation of the endogenous gene. The obtained strain was named "mutant PJC4BK" from the name of the plasmid. As stated in this article, this gene integration did not completely eliminate enzyme activity or butyrate formation due to the instability of this type of gene inactivation that can reverse to wild type by plasmid excision. This mutant strain was then used in several studies (Green and Bennett, 1998; Desai and Harris, 1999; Harris et al., 2000).

Traditionally, the commercial ABE fermentation was conducted only in a batch mode due to continuous cultures instability of the producing *Clostridia.* Several solvent yielding fermentation processes have been described. These processes yield n-butanol, acetone and ethanol in a ratio of 6:3:1. Solvent yields of 29-34% (18-25% for n-butanol only) of fermentable carbon source have been reported in the literature. A total solvent concentration of 16-24 g/I and an n-butanol concentration of 10-14 g/I is generally the limit due to toxicity of n-butanol produced. However, these low titers of solvent no longer seem to be an economical limitation to the process as it has recently been demonstrated that solvents can be recovered during fermentation by the use of the "low cost" gas striping technology.

The problem to be solved by the present invention is to obtain a stable mutant strain with no acetate formation that could be cultured for several generations without any possibility of reversion to the wild type genotype. This strain would be useful for the biological production of n-butanol at high yield by genetically stable cultures of *Clostridia,* from an inexpensive carbon substrate such as glucose or other sugars. The number of biochemical steps to inactivate and the complexity of the regulation of the metabolism necessitate the use of a metabolically engineered whole cell catalyst, for an industrial feasible process of n-butanol production.

### SUMMARY OF THE INVENTION

Applicants have solved the stated problem and the present invention provides a method for the bioconvertion of a fermentable carbon source to n-butanol as a major product by genetically stable cultures of *Clostridia.* Glucose is used as a model substrate and recombinant *Clostridium acetobutylicum* is used as the model host. In one aspect of this invention, a stable recombinant *C. acetobutylicum* unable to produce acetate is constructed by interrupting the genes coding for the phosphotransacetylase and/or acetate kinase (*pta* and *ack*). Furthermore, a recombinant *C. acetobutylicum* unable to metabolize butyryl coA into butyrate is constructed by interrupting the gene coding for the butyrate kinase (*buk*) and/or the gene *ptb.* In another aspect of this invention, a recombinant *C. acetobutylicum* unable to produce acetone is constructed by interrupting the genes coding for the CoA-transferase (*ctfAB*) and/or for aceto-acetate decarboxylase (*adc*). In a further aspect of this invention a recombinant strain unable to produce lactate is constructed by interrupting the gene coding for the lactate dehydrogenase (*ldh*). In a final aspect of this invention, the flux of hydrogen production is decreased and then the flux of reducing equivalent redirected toward n-butanol production by the interruption the gene encoding the hydrogenase *hydA.*

The present invention may be generally applied to include any carbon substrate that is readily converted to acetyl-coA.

Accordingly it is an object of the present invention to provide a recombinant organism, useful for the production of n-butanol comprising: (a) at least an interruption of one of the two genes involved in the acetate formation, (b) at least an interruption of one of the two genes involved in the conversion of butyryl-CoA to butyrate and (c) at least an interruption of one of the two genes encoding the CoA-transferase activity. Optionally the recombinant organism may comprise i) inactivating mutations in endogenous genes selected from the group consisting of:
(a) a gene encoding a polypeptide having lactate dehydrogenase activity
(b) attenuation in a gene encoding a polypeptide having hydrogenase activity.

In another embodiment the invention provides a stable process for the production of n-butanol at high yield from a recombinant organism comprising:
(a) contacting the recombinant organism of the present invention with at least one carbon source selected from the group consisting of monosaccharides, oligosaccharides, polysaccharides, and single-carbon substrates whereby n-butanol is produced; optionally (b) recovering the n-butanol during the production and (c) purifying n-butanol from the condensate by distillation.

### DETAILED DESCRIPTION OF THE INVENTION

The invention is related to a method for the production of n-butanol by culturing a microorganism in an appropriate culture medium comprising a source of carbon, and recovery of n-butanol from the culture medium, wherein at least one gene involved in acetate formation is interrupted in the microorganism by stable insertion of a foreign DNA into said at least one gene.

As used herein the following terms may be used for interpretation of the claims and specification.

The term "microorganism" refers to all kind of unicellular organisms, including prokaryotic organisms like bacteria, and eukaryotic organisms like yeasts. Preferentially, the microorganism is selected among *Enterobacteriaceae, Bacillaceae, Streptomycetaceae, Corynebacteriaceae* and *Clostridiaceae.* More preferentially, the microorganism is a species of *Escherichia, Klebsiella, Pantoea, Salmonella Corynebacterium* or *Clostridium.* Even more preferentially, the microorganism is a *Clostridium acetobutylicum.*

The expression "appropriate culture medium" refers to a culture medium adapted for the used microorganism as it is well known by the man skilled in the art.

The term "carbon substrate" or "source of carbon" means any carbon source capable of being metabolized by a microorganism wherein the substrate contains at least one carbon atom. Authors refer particularly to renewable, inexpensive and fermentable carbon sources such as monosaccharides, oligosaccharides, polysaccharides, single-carbon substrates, and polyols such as glycerol. Single carbon substrates are defined as carbon molecules that contain only one carbon atom such as methanol.

Monosaccharides of the formula (CH₂O)ₙ are also called oses or "simple sugars"; monosaccharides include saccharose, fructose, glucose, galactose and mannose. Other carbon sources comprising more than one monosaccharide are called disaccharides, trisaccharides, oligosaccharides and polysaccharides. Disaccharides include saccharose (sucrose), lactose and maltose. Starch and hemicellulose are polysaccharides, also known as "complex sugars".

Therefore, the term "source of carbon" means any product cited above, and mixture thereof.

The term "interruption" refers to the stable insertion of a foreign DNA into the gene, leading to the inactivation of the protein production, product of the gene. The foreign DNA is preferably an intron of a size appropriate to provide stabilization of the inactivation over the successive generations.

This technology is described precisely in patent applications EP 0 851 940 and EP 0 941 338, and has been used by the applicant as shown in the following examples. The term "Intron II" as used in the examples designates sequences of 950bp of bacterial mobile group II intron, originating from the Lactococcus lactis L1.LtrB intron.

In the description of the present invention, enzymes are identified by their specific activities. This definition thus includes all polypeptides that have the defined specific activity also present in other organisms, more particularly in other microorganisms. Often enzymes with similar activities can be identified by their grouping to certain families defined as PFAM or COG.

PFAM (protein families database of alignments and hidden Markov models; http://www.sanger.ac.uk/Software/Pfam/) represents a large collection of protein sequence alignments. Each PFAM makes it possible to visualize multiple alignments, see protein domains, evaluate distribution among organisms, gain access to other databases, and visualize known protein structures.

COGs (clusters of orthologous groups of proteins; http://www.ncbi.nlm.nih.gov/COG/) are obtained by comparing protein sequences from 43 fully sequenced genomes representing 30 major phylogenic lines. Each COG is defined from at least three lines, which permits the identification of former conserved domains.

The means of identifying homologous sequences and their percentage homologies are well known to those skilled in the art, and include in particular the BLAST programs, which can be used from the website http://www.nchi.nlm.nih.gov/BLAST/ with the default parameters indicated on that website. The sequences obtained can then be exploited (e.g., aligned) using, for example, the programs CLUSTALW (http://www.ebi.ac.uk/clustalw/) or MULTALIN (http://prodes.toulouse.inra.fr/multalin/cgi-bin/multalin.pl), with the default parameters indicated on those websites.

Using the references given on GenBank for known genes, those skilled in the art are able to determine the equivalent genes in other organisms, bacterial strains, yeasts, fungi, mammals, plants, etc. This routine work is advantageously done using consensus sequences that can be determined by carrying out sequence alignments with genes derived from other microorganisms, and designing degenerate probes to clone the corresponding gene in another organism. These routine methods of molecular biology are well known to those skilled in the art, and are described, for example, in Sambrook et al. (Molecular Cloning: a Laboratory Manual. 2nd ed. Cold Spring Harbor Lab., Cold Spring Harbor, New York, 1989.).

The present invention provides a method for the fermentative batch or continuous production of n-butanol by culturing a microorganism in an appropriate culture medium comprising a carbon source and the recovery of n-butanol from the culture medium wherein at least one gene involved in acetate formation is deleted in the microorganism. Preferentially, the recovery of butanol is simultaneous to the culture.

A specific embodiment of the invention provides a method wherein the microorganism is modified to be unable to produce acetate due to the interruption of at least one gene encoding for phospho-transacetylase (*pta*) or acetate kinase (*ack*).

Interruption of genes in *Clostridia* can be done using the method described in patent applications EP 0 851 940 and EP 0 941 338.

In another embodiment of the invention, the microorganism is modified to be unable to produce butyrate. In a preferred embodiment, the microorganism is unable to convert butyryl-CoA to butyrate due to the interruption of at least one gene involved in butyrate formation, in particular a gene selected from the following: genes encoding for phospho-transbutyrylase (*ptb*) or butyrate kinase (*buk*).

In another embodiment of the invention, the microorganism is unable to produce acetone. Preferentially in said microorganism, at least one gene involved in acetone formation is interrupted in the microorganism.

In a preferred embodiment, this inability to produce acetone is due to an interruption of at least one of the gene encoding for CoA-transferase (*ctfAB*) or acetoacetate decarboxylase (*adc*).

In a further embodiment of the invention, the microorganism used in the method of the invention is unable to produce lactate. In particular this can be due to an interruption of the gene *ldh* encoding for lactate dehydrogenase.

An embodiment of the invention also provides a microorganism as described above, with a decreased flux of hydrogen production and then a redirection of the flux of reducing equivalent toward n-butanol production; this can be done by interrupting the gene encoding the hydrogenase (*hydA*), an enzyme that provides a sink for reducing equivalent in the form of hydrogen production.

According to the invention, the interruption of genes is performed by insertion of an intron into the gene.

Preferably, the used microorganism is selected among the group consisting of *C. acetobutylicum, C. beijerinckii, C. saccharoperbutylacetonicum* or *C. saccharobutylicum.*

In another embodiment of the invention, the culture is continuous and stable.

In another embodiment, the method according to the invention comprises the following steps:
(a) Fermentation of a microorganism producing butanol, by contacting said microorganism with at least one carbon source selected from the group consisting of glucose, xylose, arabinose, sucrose, monosaccharides, oligosaccharides, polysaccharides, cellulose, xylan, starch or its derivatives and glycerol,
(b) Optionally, recovering the n-butanol during the fermentation and
(c) Isolation of n-butanol from the condensate by distillation.

Those skilled in the art are able to define the culture conditions for the microorganisms according to the invention. In particular the clostridia are fermented at a temperature between 20°C and 55°C, preferentially between 25°C and 40°C, and more specifically about 35°C for *C. acetobutylicum.*

The fermentation is generally conducted in fermentors with an inorganic culture medium of known defined composition adapted to the bacteria used, containing at least one simple carbon source, and if necessary a co-substrate necessary for the production of the metabolite.

The invention is also related to the microorganism as described previously. Preferably, this microorganism is selected among the group consisting of *C. acetobutylicum, C. beijerinckii, C. saccharoperbutylacetonicum* or *C. saccharobutylicum.*

### DESCRIPTION OF DRAWINGS

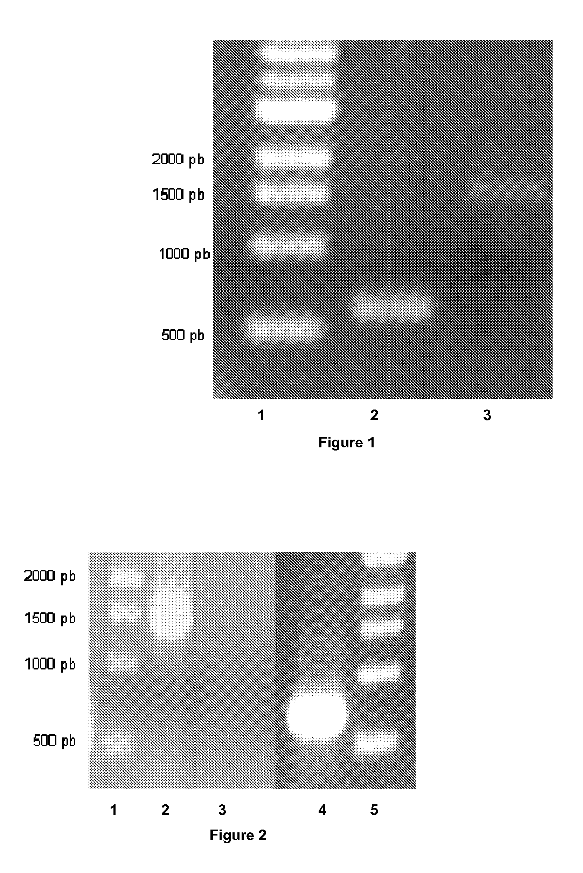

**Figure 1****:** Agarose gel of the interruption of the *pta* gene with an intron in sens orientation. Line 1, molecular marker; line 2, *pta* amplification in the wild type strain (603 pb); line 3, *pta* gene interrupted in sens orientation by the Intron (1552 pb).
**Figure 2****:** Agarose gel of the interruption of the *ptb* gene with an intron in sens orientation. Line 1, molecular marker; line 2, *ptb* gene interrupted in sens orientation by the Intron (1588 pb); line 3, empty lane; line 4, *ptb* amplification in the wild type strain (639 pb); line 5, molecular marker. The sizes of the molecular marker bands are indicated.

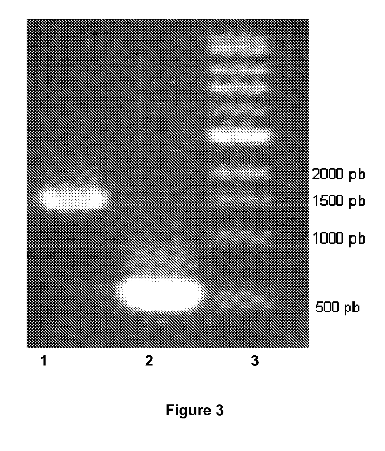

**Figure 3****:** Agarose gel of the interruption of the *hydA* gene with an intron in sens orientation. Line 1, *hydA* gene interrupted in sens orientation by the Intron (1438 pb); line 2, *hydA* amplification in the wild type strain (488 pb); line 3, molecular marker. The sizes of the molecular marker bands are indicated.

### EXAMPLES

### EXAMPLE 1

### Contruction of the pCONS:: upp-intron vector

This plasmid contains a pIM13 origin of replication fuctional in Clostridia, a *catP* gene conferring resistance to thiamphenicol, the *upp* gene and LtrA ORF required for functional expression of the group II intron RNP. In order to construct the pCONS-intron vector, we sub-cloned the sequence of the LtrA ORF into the pCONS::*upp* vector. The LtrA ORF region was obtained from restriction digestion of pACD4 vector (Sigma TargeTron) with *XbaI* and *PshAI.* The pSOS95 vector was digested with *BamHI* and *SfoI* blunt ended to remove the acetone formation genes while leaving the thiolase promoter region. The LtrA ORF digest product and the linearized pSOS95 vector were ligated to create the pSOS-intron vector. The pSOS-intron vector was digested by *NsiI* and *SapI* to remove the thiolase promoter and LtrA ORF. The pCONS::*upp* was digested with *SapI* and blunt ended. These fragments were ligated together to generate the pCONS::*upp*-intron vector.

### EXAMPLE 2

### Contruction of the pCONS:: upp-intron ack sense and pCONS:: upp-intron ack antisense vectors

To inactivate the *ack* gene, a strain with the insertion of sense or antisense intron II in the *ack* gene was constructed as follows. First, a computer algorithm was used to identify target sites in the *ack* gene. Second, the computer algorithm outputs primer sequences (Table 1) which are used to mutate (re-target) the *ack* sense intron by PCR with the primers ACK 1, ACK 2, ACK 3 and the EBS universal primer or the *ack* antisense intron by PCR with the primers ACK 4, ACK 5, ACK 6 and the EBS universal primer. Next, the mutated 350 pb PCR fragment *ack* sense or antisense intron and the pCONS::*upp*-intron vector were digested by *BsrGI* and *HindIII* and then ligated to yield the pCONS::*upp*-intron *ack* sense or the pCONS::*upp*-intron *ack* antisense.

**Table 1: primers sequences**

| Name | | Primer sequences |
|---|---|---|
| ACK 1(IBS) | SEQ ID N°1 | AAAAAAGCTTATAATTATCCTTAGTACTCGCTAAAGTGCGC CCAGATAGGGTG |
| ACK 2 (EBS1d) | SEQ ID N°2 | CAGATTGTACAAATGTGGTGATAACAGATAAGTCGCTAAAG GTAACTTACCTTTCTTTGT |
| ACK 3 (EBS2) | SEQ ID N°3 | TGAACGCAAGTTTCTAATTTCGATTAGTACTCGATAGAGGA AAGTGTCT |
| ACK 4(IBS) | SEQ ID N°4 | AAAAAAGCTTATAATTATCCTTATCTAACACAAGCGTGCGC CCAGATAGGGTG |
| ACK 5 (EBS1d) | SEQ ID N°5 | CAGATTGTACAAATGTGGTGATAACAGATAAGTCACAAGCT TTAACTTACCTTTCTTTGT |
| ACK 6 (EBS2) | SEQ ID N°6 | TGAACGCAAGTTTCTAATTTCGGTTTTAGATCGATAGAGGA AAGTGTCT |

### EXAMPLE 3

### Contruction of the pCON ::upp-intron pta sense and pCONS::upp-intron pta antisense vectors

To inactivate the *pta* gene, a strain with the insertion of sense or antisense intron II in the *pta* gene was constructed as follows. First, a computer algorithm was used to identify target sites in the *pta* gene. Second, the computer algorithm outputs primer sequences (Table 2) which are used to mutate (re-target) the *pta* sense intron by PCR with the primers PTA 1, PTA 2, PTA 3 and the EBS universal primer or the *pta* antisense intron by PCR with the primers PTA 4, PTA 5, PTA 6 and the EBS universal primer. Next, the mutated 350 pb PCR fragment *pta* sense or antisense intron and the pCONS::*upp*-intron vector were digested by *BsrGI* and *HindIII* and then ligated to yield the pCONS::*upp*-intron *pta* sense or the pCONS::upp-intron *pta* antisense.

**Table 2: primers sequences**

| Name | | Primer sequences |
|---|---|---|
| PTA 1(IBS) | SEQ ID N°7 | AAAAAAGCTTATAATTATCCTTAGAAGACAAAAGAGTGCGC CCAGATAGGGTG |
| PTA 2 (EBS1d) | SEQ ID N°8 | CAGATTGTACAAATGTGGTGATAACAGATAAGTCAAAAGAA ATAACTTACCTTTCTTTGT |
| PTA 3 (EBS2) | SEQ ID N°9 | TGAACGCAAGTTTCTAATTTCGATTTCTTCTCGATAGAGGA AAGTGTCT |
| PTA 4(IBS) | SEQ ID N°10 | AAAAAAGCTTATAATTATCCTTATGTACCCAATGTGTGCGC CCAGATAGGGTG |
| PTA 5 (EBS1d) | SEQ ID N°11 | CAGATTGTACAAATGTGGTGATAACAGATAAGTCCAATGTT TTAACTTACCTTTCTTTGT |
| PTA 6 (EBS2) | SEQ ID N°12 | TGAACGCAAGTTTCTAATTTCGGTTATACATCGATAGAGGA AAGTGTCT |

### EXAMPLE 4

### Contruction of the pCONS::upp-intron buk sense and pCONS::upp-intron buk antisense vectors

To inactivate the *buk* gene, a strain with the insertion of sense or antisense intron II in the *buk* gene was constructed as follows. First, a computer algorithm was used to identify target sites in the *buk* gene. Second, the computer algorithm outputs primer sequences (Table 3) which are used to mutate (re-target) the *buk* sense intron by PCR with the primers BUK 1, BUK 2, BUK 3 and the EBS universal primer or the *buk* antisense intron by PCR with the primers BUK 4, BUK 5, BUK 6 and the EBS universal primer. Next, the mutated 350 pb PCR fragment *buk* sense or antisense intron and the pCONS::*upp*-intron vector were digested by *BsrGI* and *HindIII* and then ligated to yield the pCONS::*upp*-intron *buk* sense or the pCONS::upp-intron *buk* antisense.

**Table 3: primers sequences**

| Name | | Primer sequences |
|---|---|---|
| BUK 1(IBS) | SEQ ID N°13 | AAAAAAGCTTATAATTATCCTTACAACTCAAATTGGTGCGC CCAGATAGGGTG |
| BUK 2 (EBS1d) | SEQ ID N°14 | CAGATTGTACAAATGTGGTGATAACAGATAAGTCAAATTG GTTAACTTACCTTTCTTTGT |
| BUK 3 (EBS2) | SEQ ID N°15 | TGAACGCAAGTTTCTAATTTCGGTTAGTTGTCGATAGAGG AAAGTGTCT |
| BUK 4(IBS) | SEQ ID N°16 | AAAAAAGCTTATAATTATCCTTATGTATCCTGGAAGTGCGC CCAGATAGGGTG |
| BUK 5 (EBS1d) | SEQ ID N°17 | CAGATTGTACAAATGTGGTGATAACAGATAAGTCCTGGAA CATAACTTACCTTTCTTTGT |
| BUK 6 (EBS2) | SEQ ID N°18 | TGAACGCAAGTTTCTAATTTCGGTTATACATCGATAGAGG AAAGTGTCT |

### EXAMPLE 5

### Contruction of the pCONS::upp-intron ptb sense vector

To inactivate the *ptb* gene, a strain with the insertion of sense intron II in the *ptb* gene was constructed as follows. First, a computer algorithm was used to identify target sites in the *ptb* gene. Second, the computer algorithm outputs primer sequences (Table 4) which are used to mutate (re-target) the *ptb* sense intron by PCR with the primers PTB 1, PTB 2, PTB 3 and the EBS universal primer. Next, the mutated 350 pb PCR fragment ptb sense intron and the pCONS::*upp*-intron vector were digested by *BsrGI* and *HindIII* and then ligated to yield the pCONS::upp-intron *ptb* sense.

**Table 4: primers sequences**

| Name | | Primer sequences |
|---|---|---|
| PTB 1(IBS) | SEQ ID N°19 | AAAAAAGCTTATAATTATCCTTACAAGACGAGCCAGTGC GCCCAGATAGGGTG |
| PTB 2 (EBS1d) | SEQ ID N°20 | CAGATTGTACAAATGTGGTGATAACAGATAAGTCGAGCC AGTTAACTTACCTTTCTTTGT |
| PTB 3 (EBS2) | SEQ ID N°21 | TGAACGCAAGTTTCTAATTTCGGTTTCTTGTCGATAGAG GAAAGTGTCT |

### EXAMPLE 6

### Contruction of the pCONS:: upp-intron ctfA sense and pCONS:: upp-intron ctfA antisense vectors

To inactivate the *ctfA* gene, a strain with the insertion of sense or antisense intron II in the *ctfA* gene was constructed as follows. First, a computer algorithm was used to identify target sites in the *ctfA* gene. Second, the computer algorithm outputs primer sequences (Table 5) which are used to mutate (re-target) the *ctfA* sense intron by PCR with the primers CTFA 1, CTFA 2, CTFA 3 and the EBS universal primer or the *ctfA* antisense intron by PCR with the primers CTFA 4, CTFA 5, CTFA 6 and the EBS universal primer. Next, the mutated 350 pb PCR fragment *ctfA* sense or antisense intron and the pCONS::*upp*-intron vector were digested by *BsrGI* and *HindIII* and then ligated to yield the pCONS::*upp*-intron *ctfA* sense or the pCONS::*upp*-intron *ctfA* antisense.

**Table 5: primers sequences**

| Name | | Primer sequences |
|---|---|---|
| CTFA 1(IBS) | SEQ ID N°22 | AAAAAAGCTTATAATTATCCTTATAGGTCGTGTACGTG CGCCCAGATAGGGTG |
| CTFA 2 (EBS1d) | SEQ ID N°23 | CAGATTGTACAAATGTGGTGATAACAGATAAGTCGTGT ACTATAACTTACCTTTCTTTGT |
| CTFA 3 (EBS2) | SEQ ID N°24 | TGAACGCAAGTTTCTAATTTCGGTTACCTATCGATAGA GGAAAGTGTCT |
| CTFA 4(IBS) | SEQ ID N°25 | AAAAAAGCTTATAATTATCCTTAATATACGGATTAGTGC GCCCAGATAGGGTG |
| CTFA 5 (EBS1d) | SEQ ID N°26 | CAGATTGTACAAATGTGGTGATAACAGATAAGTCGGAT TAAATAACTTACCTTTCTTTGT |
| CTFA 6 (EBS2) | SEQ ID N°27 | TGAACGCAAGTTTCTAATTTCGGTTTATATCCGATAGA GGAAAGTGTCT |

### EXAMPLE 7

### Contruction of the pCONS::upp-intron ldh sense and pCONS::upp-intron ldh antisense vectors

To inactivate the *ldh* gene, a strain with the insertion of sense or antisense intron II in the *ldh* gene was constructed as follows. First, a computer algorithm was used to identify target sites in the *ldh* gene. Second, the computer algorithm outputs primer sequences (Table 6) which are used to mutate (re-target) the *ldh* sense intron by PCR with the primers LDH 1, LDH 2, LDH 3 and the EBS universal primer or the *ldh* antisense intron by PCR with the primers LDH 4, LDH 5, LDH 6 and the EBS universal primer. Next, the mutated 350 pb PCR fragment *ldh* sense or antisense intron and the pCONS::*upp*-intron vector were digested by *BsrGI* and *HindIII* and then ligated to yield the pCONS::*upp*-intron *ldh* sense or the pCONS::*upp*-intron *ldh* antisense.

**Table 6: primers sequences**

| Name | | Primer sequences |
|---|---|---|
| LDH 1(IBS) | SEQ ID N°28 | AAAAAAGCTTATAATTATCCTTACTTGCCTCTGAGGTACG CCCAGATAGGGTG |
| LDH 2 (EBS1d) | SEQ ID N°29 | CAGATTGTACAAATGTGGTGATAACAGATAAGTCTCTGAG ATTAACTTACCTTTCTTTGT |
| LDH 3 (EBS2) | SEQ ID N°30 | TGAACGCAAGTTTCTAATTTCGGTTGCAAGTCGATAGAGG AAAGTGTCT |
| LDH 4(IBS) | SEQ ID N°31 | AAAAAAGCTTATAATTATCCTTACCCATCTACACGGTGCG CCCAGATAGGGTG |
| LDH 5 (EBS1d) | SEQ ID N°32 | CAGATTGTACAAATGTGGTGATAACAGATAAGTCTACACG TCTAACTTACCTTTCTTTGT |
| LDH 6 (EBS2) | SEQ ID N°33 | TGAACGCAAGTTTCTAATTTCGGTTATGGGTCGATAGAGG AAAGTGTCT |

### EXAMPLE 8

### Contruction of the pCONS::upp-intron hydA sense and pCONS:: upp-intron hydA antisense vectors

To inactivate the *hydA* gene, a strain with the insertion of sense or antisense intron II in the *hydA* gene was constructed as follows. First, a computer algorithm was used to identify target sites in the *hydA* gene. Second, the computer algorithm outputs primer sequences (Table 7) which are used to mutate (re-target) the *hydA* sense intron by PCR with the primers HYDA 1, HYDA 2, HYDA 3 and the EBS universal primer or the *hydA* antisense intron by PCR with the primers HYDA 4, HYDA 5, HYDA 6 and the EBS universal primer. Next, the mutated 350 pb PCR fragment *hydA* sense or antisense intron and the pCONS::*upp*-intron vector were digested by *BsrGI* and *HindIII* and then ligated to yield the pCONS::*upp*-intron *hydA* sense or the pCONS::*upp*-intron *hydA* antisense.

**Table 7: primers sequences**

| Name | | Primer sequences |
|---|---|---|
| HYDA 1(IBS) | SEQ ID N°34 | AAAAAAGCTTATAATTATCCTTAATTACCATCCTTGTGC GCCCAGATCGGGTG |
| HYDA 2 (EBS1d) | SEQ ID N°35 | CAGATTGTACAAATGTGGTGATAACAGATAAGTCATCC TTGATAACTTACCTTTCTTTGT |
| HYDA 3 (EBS2) | SEQ ID N°36 | TGAACGCAAGTTTCTAATTTCGGTTGTAATCCGATAGA GGAAAGTGTCT |
| HYDA 4(IBS) | SEQ ID N°37 | AAAAAAGCTTATAATTATCCTTATATCTCCTGGTAGTGC GCCCAGATAGGGTG |
| HYDA 5 (EBS1d) | SEQ ID N°38 | CAGATTGTACAAATGTGGTGATAACAGATAAGTCCTGG TAAATAACTTACCTTTCTTTGT |
| HYDA 6 (EBS2) | SEQ ID N°39 | TGAACGCAAGTTTCTAATTTCGGTTAGATATCGATAGAG GAAAGTGTCT |

### EXAMPLE 9

### Construction of strains unable to produce acetate: Clostridium acetobutylicum Δcac1515 Δupp pta::intronII

The pCONS::upp-intron *pta* sense plasmid was used to transform by electroporation *C. acetobutylicum* MGC Δ*cac*15 Δ*upp* strain. After selection on Petri plate for clones resistant to thiamphenicol (50 µg/ml), one colony was cultured for 24 hours in liquid synthetic medium with thiamphenicol at 50 µg/ml and 100 µl of undiluted culture was plated on RCA with thiamphenicol at 50 µg/ml. The insertion of the intron II in clones resistant to thiamphenicol was checked by PCR analysis with primers PTA 7 and PTA 8 (Table 8) located outside of the *pta* gene.

One colony of the *C. acetobutylicum pta*::intron II pCONS::upp-intron pta sense strain was then cultured in chemostat on synthetic medium without antibiotic. Serial dilution of the culture was regularly plated on 2YTG plates containing 5-Fluorouracyl (5-FU). Several clones were obtained which were resistant to 5-FU. When transferred on 2YTG plates plus or minus thiamphenicol, a large number of these clones did show thiamphenicol resistance, suggesting they still contained the pCONS::*upp*-intron *pta* sense with a mutation in the *upp* gene, conferring resistance to 5-FU. However, some clones could be shown to be thiamphenicol sensitive. We could confirm that these last clones did not contain the pCONS::*upp*-intron *pta* sense plasmid but still had the insertion of the intron II in the *pta* gene (Figure 1). We could therefore easily select for a complementary mutation allowing for the inactivation of the phosphotransacetylase in this strain.

**Table 8: primers sequences**

| | | |
|---|---|---|
| PTA 7 | SEQ ID N°40 | ATTAAATGCAAACTTAAATGCAATTTTTA |
| PTA 8 | SEQ ID N°41 | GTTGTGTGAACAGCTCCTGAAACCATTCC |

### EXAMPLE 10

### Construction of strains unable to produce acetate and butyrate: C. acetobutylicum Δcac1515 Δupp ack::intronII ptb::intronII

The pCONS::upp-intron ptb sense plasmid was used to transform by electroporation *C. acetobutylicum* MGC Δ*cac*15 Δ*upp ack*::intron II strain. After selection on Petri plate for clones resistant to thiamphenicol (50 µg/ml), one colony was cultured for 24 hours in liquid synthetic medium with thiamphenicol at 50 µg/ml and 100 µl of undiluted culture was plated on RCA with thiamphenicol at 50 µg/ml. The insertion of the intron II in clones resistant to thiamphenicol was checked by PCR analysis with primers PTB 7 and PTB 8 (Table 9) located outside of the *ptb* gene (Figure 2). The *C. acetobutylicum* Δ*cac*15 Δ*upp ack::*intron II *ptb*::intron strain which has lost pCONS::*upp*-intron *ptb* sense was isolated on RCA with 5-FU at 400 µM.

**Table 9: primers sequences**

| | | |
|---|---|---|
| PTB 7 | SEQ ID N°42 | GTCTATCAAATTTCTCAGTTTCAAATACTG |
| PTB 8 | SEQ ID N°43 | GAAAGTATTTCACTAAAAGGACTATATCC |

### EXAMPLE 11

### Construction of strains unable to produce acetate, butyrate and acetone: C. acetobutylicum Δcac1515 Δupp ack::intronII ptb::intronII ctfA::intronII

The pCONS::*upp*-intron *ctfA* sense plasmid was used to transform by electroporation *C. acetobutylicum* MGC Δ*cac*15 Δ*upp ack*::intron II *ptb*::intron II strain. After selection on Petri plate for clones resistant to thiamphenicol (50 µg/ml), one colony was cultured for 24 hours in liquid synthetic medium with thiamphenicol at 50 µg/ml and 100 µl of undiluted culture was plated on RCA with thiamphenicol at 50 µg/ml. The insertion of the intron II in clones resistant to thiamphenicol was checked by PCR analysis with primers CTFA 7 and CTFA 8 (Table 10) located outside of the *ctfA* gene. The *C. acetobutylicum* Δ*cac*15 Δ*upp ack*::intron II *ptb*::intron II *ctfA*::intron II strain which has lost pCONS::*upp*-intron *ctfA* sense was isolated on RCA with 5-FU at 400 µM.

**Table 10: primers sequences**

| | | |
|---|---|---|
| CTFA 7 | SEQ ID N°44 | GCTTCATATATAGGCAGCAACCCAGATACTGGC |
| CTFA 8 | SEQ ID N°45 | CCACCCATACCAGAGAGCATTTTTCCAGG |

### EXAMPLE 12

### Construction of strains unable to produce acetate, butyrate, acetone and lactate: C. acetobutylicum Δcac1515 Δupp ack::intronII ptb::intronII ctfA::intronII ldh::intronII

The pCONS::*upp*-intron *ldh* sense plasmid was used to transform by electroporation *C. acetobutylicum* MGC Δ*cac*15 Δ*upp ack*::intron II *ptb*::intron II *ctfA*::intron II strain. After selection on Petri plate for clones resistant to thiamphenicol (50 µg/ml), one colony was cultured for 24 hours in liquid synthetic medium with thiamphenicol at 50 µg/ml and 100 µl of undiluted culture was plated on RCA with thiamphenicol at 50 µg/ml. The insertion of the intron II in clones resistant to thiamphenicol was checked by PCR analysis with primers LDH 7 and LDH 8 (Table 11) located outside of the *ldh* gene. The *C. acetobutylicum* Δ*cac*15 Δ*upp ack*::intron II *ptb*::intron II *ctfA*::intron II *ldh*::intron II strain which has lost pCONS::*upp*-intron *ldh* sense was isolated on RCA with 5-FU at 400 µM.

**Table 11: primers sequences**

| | | |
|---|---|---|
| LDH 7 | SEQ ID N°46 | GGAACCTCTATAATATCCTTCACTCCGTTAATTCC |
| LDH 8 | SEQ ID N°47 | GGATTTGTTGGTTCTTCTACAGTATTTGCGC |

### EXAMPLE 13

### Construction of strains with lower hydrogen production: C. acetobutylicum Δcac1515 Δupp hydA::intronII

The pCONS::*upp*-intron *hydA* sense plasmid was used to transform by electroporation *C. acetobutylicum* MGC Δ*cac*15 Δ*upp* strain. After selection on Petri plate for clones resistant to thiamphenicol (50 µg/ml), one colony was cultured for 24 hours in liquid synthetic medium with thiamphenicol at 50 µg/ml and 100 µl of undiluted culture was plated on RCA with thiamphenicol at 50 µg/ml. The insertion of the intron II in clones resistant to thiamphenicol was checked by PCR analysis (with primers HYDA 7 and HYDA 8 (Table 12) located outside of the *hydA* gene.

One colony of the *C. acetobutylicum hydA*::intron II pCONS::upp-intron hydA sense strain was then cultured in chemostat on synthetic medium without antibiotic. Serial dilution of the culture was regularly plated on 2YTG plates containing 5-Fluorouracyl (5-FU). Several clones were obtained which were resistant to 5-FU. When transferred on 2YTG plates plus or minus thiamphenicol, a large number of these clones did show thiamphenicol resistance, suggesting they still contained the pCONS::*upp*-intron *hydA* sense with a mutation in the *upp* gene, conferring resistance to 5-FU. However, some clones could be shown to be thiamphenicol sensitive. We could confirm that these last clones did not contain the pCONS::upp-intron hydA sense plasmid but still had the insertion of the intron II in the *hydA* gene (Figure 3). We could therefore easily select for a complementary mutation allowing for the inactivation of the hydrogenase in this strain.

**Table 12: primers sequences**

| | | |
|---|---|---|
| HYDA 7 | SEQ ID N°48 | GGTATTGAATATTCGAAATCAACC |
| HYDA 8 | SEQ ID N°49 | GCTGATAAATATGTAGCTGAACCTGG |

### EXAMPLE 14

### Batch fermentation of n-butanol producing strains.

Strains were initially analyzed in anaerobic flask cultures in the synthetic medium described by Soni *et al* (Soni et al, 1987, Appl. Microbiol.Biotechnol. 27:1-5) supplemented with 2.5 g/l of ammonium acetate. An overnight culture at 35°C was used to inoculate a 30 ml culture to an OD600 of 0.05. After incubation of the culture for 3 days at 35°C, glucose, organic acids and solvents were analyzed by HPLC using a Biorad HPX 97H column for the separation and a refractometer for the detection.

Strains with the correct phenotype were subsequently tested under production conditions in 300 ml fermentors (DASGIP) using an anaerobic batch protocol.

For this purpose the fermentor was filled with 250 ml of synthetic medium, sparged with nitrogen for 30 min and inoculated with 25 ml of preculture to an optical density (OD600nm) between 0.05 and 0.1.

The temperature of the culture was maintained constant at 35 °C and the pH was permanently adjusted at 5.5 using an NH₄OH solution. The agitation rate was maintained at 300 rpm during the fermentation.

### EXAMPLE 15

### Continuous fermentation of n-butanol producing strains.

The best n-butanol producing strain was analyzed in chemostat cultures in the synthetic medium described by Soni *et al* (Soni et al, 1987, Appl. Microbiol.Biotechnol. 27:1-5). An overnight culture at 35°C was used to inoculate a 300 ml fermentors (DASGIP) using an anaerobic chemostat protocol.
For this purpose the fermentor was filled with 250 ml of synthetic medium, sparged with nitrogen for 30 min and inoculated with 25 ml of preculture to an optical density (OD600nm) between 0.05 and 0.1. After 12 hours of batch culture at 35 °C, pH 5.5 (regulated using an NH₄OH solution) and an agitation rate of 300 rpm, the fermentor was continuously fed with oxygen free synthetic medium at a dilution rate of 0.05 h-1 while the volume was kept constant by sequential removal of fermentated medium. Stability of the culture was followed by products analysis using the HPLC protocol previously described.

### REFERENCES

Desai RP, Harris LM, Welker NE, Papoutsakis ET. Metabolic flux analysis elucidates the importance of the acid-formation pathways in regulating solvent production by Clostridium acetobutylicum. Metab. Eng. 1999, 1: 206-13.
Green EM, Bennett GN. Genetic manipulation of acid and solvent formation in Clostridium acetobutylicum ATCC 824 Biotechnol. Bioeng. 1998, 58: 215-21.
Green EM, Boynton ZL, Harris LM, Rudolph FB, Papoutsakis ET, Bennett GN. Genetic manipulation of acid formation pathways by gene inactivation in Clostridium acetobutylicum ATCC 824. Microbiology. 1996, 142: 2079-86.
Harris LM, Desai RP, Welker NE, Papoutsakis ET. Characterization of recombinant strains of the Clostridium acetobutylicum butyrate kinase inactivation mutant: need for new phenomenological models for solventogenesis and butanol inhibition? Biotechnol. Bioeng. 2000, 67: 1-11.
Soni B. K., Soucaille P. Goma G. Continuous acetone butanol fermentation: influence of vitamins on the metabolic activity of Clostridium acetobutylicum. Appl. Microbiol. Biotechnol. 1987, 27: 1-5.

## Claims

1. A method for the production of n-butanol by culturing a microorganism in an appropriate culture medium comprising a source of carbon and recovery of n-butanol from the culture medium, wherein at least one gene involved in acetate formation is interrupted in the microorganism by stable insertion of a foreign DNA into said at least one gene.

2. The method according to claim 1 wherein the interrupted gene is at least one of the following genes:
• *pta* encoding phospho- transacetylase
• *ack* encoding acetate kinase.

3. The method as claimed in any one of claims 1 or 2 wherein the microorganism is modified to be unable to produce butyrate.

4. The method as claimed in claim 3 in which at least one gene involved in butyrate formation is interrupted.

5. The method according to claim 4 wherein the interrupted gene is selected among the following :
• *ptb* encoding phospho-transbutyrylase
• *buk* encoding butyrate kinase.

6. The method according to any one of claims 1 to 5 wherein at least one gene involved in acetone formation is interrupted in the microorganism.

7. The method according to claim 6 wherein at least one of the following genes is interrupted:
• *ctfAB* encoding CoA-transferase
• *adc* encoding aceto-acetate decarboxylase.

8. The method according to any one of claims 1 to 7 wherein the microorganism is modified to be unable to produce lactate.

9. The method according to claim 8 wherein the *ldh* gene is interrupted.

10. The method as claimed in any one of claims 1 to 9, wherein the hydrogen flux is decreased and the reducing power redirected to butanol production.

11. The method as claimed in claim 10 wherein the *hydA* gene is interrupted.

12. The method as claimed in any one of claims 1 to 11, wherein the at least one gene is interrupted by insertion of an intron into the gene.

13. The method according to anyone of claims 1 to 12 wherein the microorganism is selected among the group consisting of *C. acetobutylicum, C. beijerinckii, C. saccharoperbutylacetonicum* or *C. saccharobutylicum.*

14. The method according to anyone of claims 1 to 13 wherein the culture is continuous and stable.

15. The method according to claim 14 comprising the following steps:
a) Fermentation of the microorganism producing n-butanol;
b) Optionally the elimination of n-butanol during the fermentation;
c) Isolation of n-butanol from the condensate by distillation.

16. The microorganism as defined in any one of claims 1 to 13.
